# EUROPEAN PATENT APPLICATION

(11) **EP 1 637 130 A1**
(43) Date of publication of application: **22.03.2006**
(21) Application number: 04742013.8
(22) Date of filing: 16.06.2004
(51) Int. Cl.: A61K 9/50

(54) **MICROCAPSULES FOR THE ADMINISTRATION OF ACTIVE INGREDIENTS**

(30) Priority: 18.06.2003 ES 200301424
(71) Applicant: Lipofoods, S.L., 08850 Gava (ES)
(72) Inventor: MORATO RIERA, Miriam, E-08031 Barcelona (ES); PARENTE DUENA, Antonio, E-08960 Sant Just Desvern (ES); GARCES GARCES, Josep, E-08760 Martorell (ES)
(74) Representative: Carvajal y Urquijo, Isabel
(86) International application number: PCT/ES2004/000277
(87) International publication number: WO 2004/110412

(57) **Abstract**

The present invention relates to microcapsules for the administration of active ingredients, consisting of a core comprising one or more active ingredients, a surfactant and at least one wax or at least one fat, or a mixture of at least one wax and at least one fat, and of a coating comprising one or more non-fat substances selected from the group comprising polysaccharides, animal proteins, milk proteins and vegetable proteins.

## Description

### FIELD OF THE INVENTION

The present invention relates to microcapsules for the administration of active ingredients authorized for nutritional, dietetic, pharmaceutical and/or veterinary use, consisting of a core and a coating.

### STATE OF THE ART

Different types of microcapsules are known in the state of the art. Most of them are controlled active ingredient release microcapsules.

Microcapsules are known from patent document US 5,585,050, containing at least one active ingredient, consisting of an internal hydrophilic, essentially non aqueous core formed from a solution of at least one water-soluble and amphoteric active ingredient in at least one hydrophilic non-aqueous solvent, and formed of a wall enclosing the inner core, based on at least one polymer or copolymer. These microcapsules are applicable in instant active ingredient release systems, such as articles for hygiene, gloves and surgical materials.

Microcapsules are known from patent document EP 0 336 662, containing biologically active compounds with an oily base, stable for prolonged periods of time, for the release of the encapsulated compound in the intestine. An example of a compound to be incorporated in these microcapsules is fish oil with a high omega 3 polyunsaturated fatty acid content. The microcapsules allow disguising the bad taste and smell of fish oil.

Nevertheless, the microcapsules known in the state of the art are suitable only for stable active ingredients over a prolonged period of time.

It was therefore necessary to provide a method of administering active ingredients, including easily degradable and therefore rather unstable active ingredients.

### DESCRIPTION OF THE INVENTION

It has now been found that by means of coating a core with polysaccharides, animal proteins, milk proteins and vegetable proteins, comprising at least one active ingredient, a surfactant and at least one wax or at least one fat, or a mixture of at least one wax and at least one fat, the fats and waxes having melting points exceeding 45°C, active ingredients can be effectively encapsulated, including easily degradable active ingredients, particularly due to oxidation.

Therefore, according to a first aspect the invention relates to microcapsules for the administration of active ingredients for their use in human nutrition, dietetics, pharmacy and animal and/or veterinary nutrition, consisting of:
- a core comprising at least one active ingredient, a surfactant and at least one wax or at least one fat, or a mixture of at least one wax and at least one fat, suitable for nutritional, dietetic, pharmaceutical and/or veterinary use, the fats and waxes having melting points exceeding 45°C, and
- a coating comprising one or more non-fat substances, suitable for nutritional, dietetic, pharmaceutical and/or veterinary use, selected from the group comprising polysaccharides, animal proteins, milk proteins and vegetable proteins.

According to a second aspect, the invention relates to nutritional, dietetic, pharmaceutical and/or veterinary compositions incorporating the microcapsules object of the present invention.

According to a further aspect, the present invention relates to a process of obtaining the microcapsules, characterized in that it comprises the steps of:
a) heating a wax or a fat, or a mixture of waxes or a mixture of fats, or a mixture of one or more fats and one or more waxes, the fats and waxes being suitable for nutritional, dietetic, pharmaceutical or veterinary use, and the fats and waxes having melting points exceeding 45°C, higher than the melting temperature of the corresponding wax/waxes or fat/fats;
b) mixing with an active ingredient and a surfactant;
c) emulsifying in an aqueous solution of one or more non-fat substances suitable for nutritional, dietetic, pharmaceutical or veterinary use, selected from the group comprising polysaccharides, animal proteins, milk proteins and vegetable proteins;
d) cooling the emulsion to under the melting temperature of the wax/waxes or fat/fats;
e) spray-drying the previous emulsion.

According to a preferred embodiment of the microcapsules, the core has one or more coadyuvants selected from the group comprising antioxidants and vegetable extracts and oils authorized for nutritional, dietetic, pharmaceutical and/or veterinary use.

According to a preferred embodiment, the antioxidant or antioxidants are chosen from the group comprising BHT, BHA, vitamin E acetate, vitamin C palmitate and/or rosemary essential oil.

The active ingredient incorporated in the microcapsules is a nutritional, dietetic or pharmacologically active ingredient, characterized in that it is easily degraded in preparations for nutritional, dietetic, pharmaceutical and/or veterinary use. It is preferably selected from the group comprising nutritional, dietetic, pharmaceutical and/or veterinary active ingredients that are hardly soluble or insoluble in water. It is more preferably selected from the group comprising oils of an animal or vegetable origin enriched in polyunsaturated fatty acids from the omega-3 and omega-6 groups. The microcapsules according to the present invention have also proven to be very useful for the incorporation of active ingredients selected from ascorbyl palmitate, carotenoids such as lycopene, lutein or zeaxanthin, fat-soluble vitamins, peptides, peptides with antibiotic activity, such as zinc bacitracin, enzymes, reduced iron, omega-9 oils, olive oil, linseed oil, nut oil, conjugated linoleic acids and fat-soluble polyphenols for example.

According to another preferred embodiment, the core incorporates Loders Croklaan's Revel A® as the only fat.

According to an additional preferred embodiment, the core incorporates carnauba wax or candelilla wax as the only wax.

According to another preferred embodiment of the microcapsules, the core surfactant is selected from polysorbate-60 and sodium oleate.

According to another preferred embodiment, the antioxidant or antioxidants authorized for nutritional, dietetic and/or pharmaceutical use are selected from the group comprising BHT, BHA, vitamin E acetate, vitamin C, palmitate and/or rosemary essential oil.

The present invention is further described below on the basis of a series of examples not meant to limit the invention.

### EXAMPLES

### Example 1

47 g of vegetable fat with a melting point of 62°C are melted in a suitable container and mixed with 30 g of fish oil rich in oils from the omega-3 series, 7 g of sodium oleate, 1 g of vitamin E acetate, 1 g of vitamin C palmitate and 1 g of rosemary oil extract. 100 mL of hot deionized water at a temperature slightly exceeding that of the melted fat are added and stirred vigorously, maintaining the temperature until obtaining an emulsion without lumps.

In another container 17 g of beta-lactoglobulin are dissolved in 100 mL of water and heated at 65°C. Solution A is emulsified in this beta-lactoglobulin solution without allowing the latter to cool.

The resulting emulsion is cooled under stirring to room temperature so as to achieve the solidification of the emulsion micelles, and once this temperature is reached it is subjected to a spray-drying process so as to obtain a microcapsule powder. The spraying conditions are adjusted so as to prevent, at all times, the obtained product from exceeding the melting temperature of the fat or wax used in the preparation of solution A.

### Example 2

47 g of vegetable fat with a melting point of 62°C are melted in a suitable container and mixed with 30 g of fish oil rich in oils from the omega-3 series, 7 g of sodium oleate, 1 g of vitamin E acetate, 1 g of vitamin C palmitate and 1 g of rosemary oil extract. 100 mL of deionized water at 70°C are added and stirred vigorously, maintaining the temperature until obtaining an emulsion without lumps. It is allowed to cool to a temperature of less than 50°C. 17 g of egg albumin are dissolved in the mixture.

The resulting emulsion is cooled under stirring to room temperature so as to achieve the solidification of the emulsion micelles, and once this temperature is reached it is subjected to a spray-drying process so as to obtain a microcapsule powder. The spraying conditions are adjusted so as to prevent, at all times, the obtained product from exceeding the melting temperature of the fat or wax used in the preparation of solution A.

### Example 3

50 g of carnauba wax are melted in a suitable container and mixed with 30 g of fish oil rich in oils from the omega-3 series, 7 g of sodium oleate, 1 g of vitamin E acetate, 1 g of vitamin C palmitate and 1 g of rosemary oil extract. 100 mL of deionized water at 90°C are added and stirred vigorously, maintaining the temperature until obtaining an emulsion without lumps. It is allowed to cool to a temperature of less than 50°C. 17 g of beta-lactalbumin are added.

The resulting emulsion is cooled under stirring to room temperature so as to achieve the solidification of the emulsion micelles, and once this temperature is reached it is subjected to a spray-drying process so as to obtain a microcapsule powder. The spraying conditions are adjusted so as to prevent, at all times, the obtained product from exceeding the melting temperature of the fat or wax used in the preparation of solution A.

### Example 4

The conditions for preparing the microcapsules according to the invention, depending on the fat or wax, on the coating and on the encapsulated product, are included in the following table.

| **Fat or wax** | **Coating** | **Encapsulated product** | **Emulsion temp.** | **Coating addition temp** | **Spraying temp.** | **Product collection temp.** |
|---|---|---|---|---|---|---|
| carnauba wax | corn starch | 40% 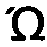 3 fish oil | 90°C | 65°C | 150°C | 65-70°C |
| carnauba wax | egg albumin | 40% 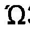 3 fish oil | 90°C | 50°C | 180°C | 65-70°C |
| carnauba wax | sodium alginate | 40% 3 fish oil | 90°C | 65°C | 120°C | 65-70°C |
| carnauba wax | beta lactalbumin | 38% 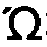 3 fish oil | 90°C | 65°C | 180°C | 65-70°C |
| candelilla wax | corn starch | 40% 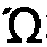 3 fish oil | 70°C | 65°C | 150°C | 45-50°C |
| vegetable fat | egg albumin | 38% 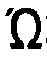 3 fish oil | 70°C | 50°C | 150°C | 45-50°C |
| vegetable fat | beta lactalbumin | 38% 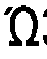 3 fish oil | 70°C | 65°C | 150°C | 45-50°C |
| vegetable fat | beta lactalbumin | 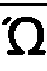 6-rich linseed oil | 70°C | 65°C | 150°C | 45-50°C |

## Claims

1. Microcapsules for the administration of active ingredients, **characterized in that** they consist of :
- a core comprising one or more active ingredient, a surfactant and at least one wax or at least one fat, or a mixture of at least one wax and at least one fat, the fats and waxes having melting points exceeding 45°C, and
- a coating comprising one or more non-fat substances selected from the group comprising polysaccharides, animal proteins, milk proteins and vegetable proteins.

2. Microcapsules according to claim 1, **characterized in that** the core contains one or more coadyuvants selected from the group comprising antioxidants and vegetable extracts and oils.

3. Microcapsules according to any of claims 1 and 2, **characterized in that** the active ingredient is a nutritional, dietetic or pharmacologically active ingredient that is easily degradable.

4. Microcapsules according to any of claims 1 to 3, **characterized in that** the active ingredient is selected form the group comprising polyunsaturated oils of a vegetable or animal origin.

5. Microcapsules according to claim 4, **characterized in that** the polyunsaturated oils are selected from the group comprising omega-3 and omega-6 oils.

6. Microcapsules according to any of claims 1 to 3, **characterized in that** the active ingredient is selected from ascorbyl palmitate, carotenoids, fat-soluble vitamins, peptides, enzymes, reduced iron, omega-9 oils, olive oil, linseed oil, nut oil, conjugated linoleic acids and fat-soluble polyphenols.

7. Microcapsules according to claim 6, **characterized in that** the active ingredient is a carotenoid selected from lycopene, lutein and zeaxanthin.

8. Microcapsules according to claim 6, **characterized in that** the active ingredient is a peptide with antibiotic activity.

9. Microcapsules according to any of claims 1 to 3, 6 and 8, **characterized in that** the active ingredient is zinc bacitracin.

10. Microcapsules according to any of the previous claims, **characterized in that** the core incorporates Loders Croklaan's Revel A® as the only fat.

11. Microcapsules according to any of the previous claims, **characterized in that** the core incorporates carnauba wax or candelilla wax as the only wax.

12. Microcapsules according to any of the previous claims, **characterized in that** the surfactant is selected from polysorbate-60 and sodium oleate.

13. Microcapsules according to claim 2, **characterized in that** the antioxidant or antioxidants are chosen from the group comprising BHT, BHA, vitamin E acetate, vitamin C palmitate and/or rosemary essential oil.

14. A nutritional, dietetic, veterinary or pharmaceutical composition **characterized in that** it incorporates microcapsules according to claims 1 to 13.

15. A process of obtaining the microcapsules according to claims 1 to 13, **characterized in that** it comprises the steps of:
a) heating a wax or a fat, or a mixture of waxes or a mixture of fats, or a mixture of one or more fats and one or more waxes, the fats and waxes being suitable for nutritional, dietetic, pharmaceutical or veterinary use, and the fats and waxes having melting points exceeding 45 °C, higher than the melting temperature of the corresponding wax/waxes or fat/fats;
b) mixing with an active ingredient and a surfactant;
c) emulsifying in an aqueous solution of one or more non-fat substances suitable for nutritional, dietetic, pharmaceutical or veterinary use, selected from the group comprising polysaccharides, animal proteins, milk proteins and vegetable proteins;
d) cooling the emulsion to under the melting temperature of the wax/waxes or fat/fats;
e) spray-drying the previous emulsion.
